# EUROPEAN PATENT SPECIFICATION

(11) **EP 3 079 576 B1**
(45) Date of publication and mention of the grant of the patent: **17.05.2023**
(21) Application number: 14820940.6
(22) Date of filing: 27.11.2014
(51) Int. Cl.: A61B 5/055, A61B 5/30, A61B 17/00

(54) **PLANAR MRI SAFE CABLE, METHOD OF USING THE CABLE AND METHOD OF MANUFACTURING THE CABLE**
PLANARES MRT-SICHERES KABEL, VERFAHREN ZUR VERWENDUNG DES KABELS UND VERFAHREN ZUR HERSTELLUNG DES KABELS
CABLE PLANAIRE SECURE POUR L'IRM, MÉTHODE D'UTILISATION DU CÂBLE ET MÉTHODE DE FABRICATION DU CÂBLE

(30) Priority: 11.12.2013 US 201361914414 P
(43) Date of publication of application: 19.10.2016
(73) Proprietor: Koninklijke Philips N.V., 5656 AG Eindhoven (NL)
(72) Inventor: O'NEILL, Francis Patrick, NL-5656 AE Eindhoven (NL); REY, Eduardo Mario, NL-5656 AE Eindhoven (NL); FORRER, Donald Alan, NL-5656 AE Eindhoven (NL)
(74) Representative: Philips Intellectual Property & Standards
(86) International application number: PCT/IB2014/066379
(87) International publication number: WO 2015/087189

(56) References cited:
- WO-A1-2013/175457
- WO-A2-2006/031317
- US-A- 6 032 063
- US-A1- 2004 225 210
- US-A1- 2010 075 537

## Description

The following relates generally to magnetic resonance (MR) imaging. It finds particular application in conjunction with Electrocardiography (ECG) during MR imaging, and will be described with particular reference thereto. However, it will be understood that it also finds application in other usage scenarios and is not necessarily limited to the aforementioned application.

MR imaging often involves biopotential measurements such ECG monitoring during the imaging procedure. Patients can be monitored for vital signs with ECG or other monitors during MR procedures to ensure patient health. ECG monitors can be used in MR imaging as triggers or gates for image capture. For example, images can be triggered such that the heart of a subject is shown only in images in a certain phase or which compensates for heart motion artifacts in the image.

ECG monitoring uses electrodes affixed to the patient at different points on the body. The electrodes sense weak electrical signals from the body which indicate phases of the heart. The electrodes are attached to leads which transmits the sensed signals to an ECG monitor which analyses the signal and/or displays the signal visually. Each lead includes a conductive wire or trace which connects the electrode to the ECG monitor and transmits signals from the attached electrode to the ECG monitor. The number of electrodes and corresponding leads can vary which means the number of wires connecting electrodes of one patient to an ECG monitor can vary. The leads can also include leads for other biopotential measurements of the patient such as respiratory monitors and the like.

Magnetic Resonance scanners use strong magnetic fields for imaging the patient and the strong magnetic fields can induce electric currents in conductive wires such as ECG traces or wires. The magnetic fields of the scanner can induce currents on the ECG wires such that give potentially false heart rate readings or obscure ECG R-waves from waveform detection schemes. The wires are susceptible to triboelectric effects and are hypersensitive to patient movement. Cross-talk between wires can interfere with the transmitted signals. Looping of wires can add to the interference where one or more wires loop around in close proximity to the same or other lead wires. Discrete wires can be inconsistent and/or inaccurate with transmitting the ECG signals. Improperly selected materials of the wire, cabling and/or insulating material which emits protons can cause artifacts in images.

Care must be taken in selecting and configuring the materials of the cabling including the wires because of patient safety. Ferrous materials can become projectiles in the strong magnetic fields of scanners which can injure patients and/or healthcare workers. Induced currents on non-MR ECG cables in the magnetic field can cause heating of the wires which may subject the patient to burns.

Current approaches for MR cables typically use high resistance wires which are braided or twisted to reduce susceptibility to noise pickup from the MR scanner. The braided or twisted bundles of wire are then insulated. Some approaches then add a shielding to further minimize interference from the magnetic field. However, these approaches still contend with cross talk between the wires twisted or braided together and do not address problems caused with looping of wires. Additionally, the twisting or braiding of the wires, and adding components such as shielding adds to the cost of the cable.

WO 2013/175457 A1 discloses a cable for use in biopotential measurements in a magnetic resonance environment comprises a flexible plastic or polymer sheet extending as a single unitary structure from a first end to an opposite second end, and an electrically conductive trace disposed on the flexible plastic or polymer sheet and running from the first end to the opposite second end. The electrically conductive trace has sheet resistance of one ohm/square or higher, and may have a hatching or checkerboard pattern. The cable may further include an electrically insulating protective layer disposed on the substrate and covering the electrically conductive trace, an electrode disposed on the electrically conductive trace at the second end, an edge connector at the first end, or various combinations of such features.

US 2004/0225210 A1 discloses a lead set assembly which is capable of coupling electrodes to a separate device. The assembly includes two leads. The condition sense lead features two conductive paths, each being connectible to one electrode for conveying a bioelectric signal therefrom and capable of having a first noise signal induced therein by electromagnetic emanations. The noise pickup lead also features two conductive paths, each being effectively topographically aligned with one conductive path of the condition sense lead to form a noise-matched pair therewith and capable of having a second noise signal induced therein by the electromagnetic emanations. In each noise-matched pair, this enables the second noise signal to be substantially approximate to the first noise signal. The lead set assembly can thus be used to convey to the separate device (I) the bioelectric and first noise signals using the condition sense lead and (II) the second noise signals using the noise pickup lead.

US 2010/0075537 A1 discloses a connector which is provided for terminating a ribbon cable having a plurality of electrical leads. Each electrical lead includes a mating portion. The connector includes a housing having a wall and a cavity. The housing is configured to hold the mating portion of each electrical lead within the cavity. The housing includes a hole extending through the wall of the housing and communicating with the cavity. The hole exposes the mating portion of at least one electrical lead through the wall.

The following discloses a new and improved planar magnetic resonance safe cable for biopotential measurements which addresses the above referenced issues, and others.

In accordance with one aspect, a magnetic resonance (MR) safe cable according to claim 1 is presented.

In accordance with another aspect, a method of using a magnetic resonance (MR) safe cable according to claim 7 is presented.

In accordance with another aspect, a method of manufacturing a magnetic resonance (MR) safe cable according to claim 9 is presented.

One advantage is a cable which operates in a strong magnetic field that is more reliable and repeatable for biopotential measurements.

Another advantage is in reducing variation in induction of interference currents in the electrode and resistive traces from the static and gradient magnetic fields.

Another advantage is in reducing triboelectric and microphonic effects by eliminating movement between resistive traces in the cable.

Another advantage resides in the increased likelihood of induced currents equally on each lead which can then be canceled out by a common mode filter.

Another advantage resides in patient safety in a strong magnetic field.

Another advantage resides in a stable, distributed, and high resistance trace.

Another advantage resides in a cable which does not cause artifacts in MR imaging.

Still further advantages will be appreciated to those of ordinary skill in the art upon reading and understanding the following detailed description.

The invention may take form in various components and arrangements of components, and in various steps and arrangement of steps. The drawings are only for purposes of illustrating the preferred embodiments and are not to be construed as limiting the invention.
FIGURES 1A-1C schematically illustrates top, cross-sectional, and side views of an embodiment of a planar magnetic resonance safe cable for biopotential measurements.
FIGURES 2A-2B schematically illustrates top and transverse views of another embodiment of the planar magnetic resonance safe cable for biopotential measurements.
FIGURE 3 schematically illustrates an embodiment of a controlled resistance electrically conductive wire.
FIGURE 4 schematically illustrates another embodiment of the planar magnetic resonance safe cable with protective shielding.
FIGURE 5 schematically illustrates another embodiment of the planar magnetic resonance safe cable with an additional component between controlled resistance electrically conductive wires.
FIGURE 6 schematically illustrates another embodiment of the planar magnetic resonance safe cable in a cross section with an additional component adjacent the controlled resistance electrically conductive wires.
FIGURE 7 schematically illustrates another embodiment of the planar magnetic resonance safe cable with a MR scanner and ECG monitor.
FIGURE 8 flowcharts one method of manufacturing an embodiment of the planar magnetic resonance safe cable for biopotential measurements.
FIGURE 9 flowcharts one method of using an embodiment of the planar magnetic resonance safe cable for biopotential measurements.

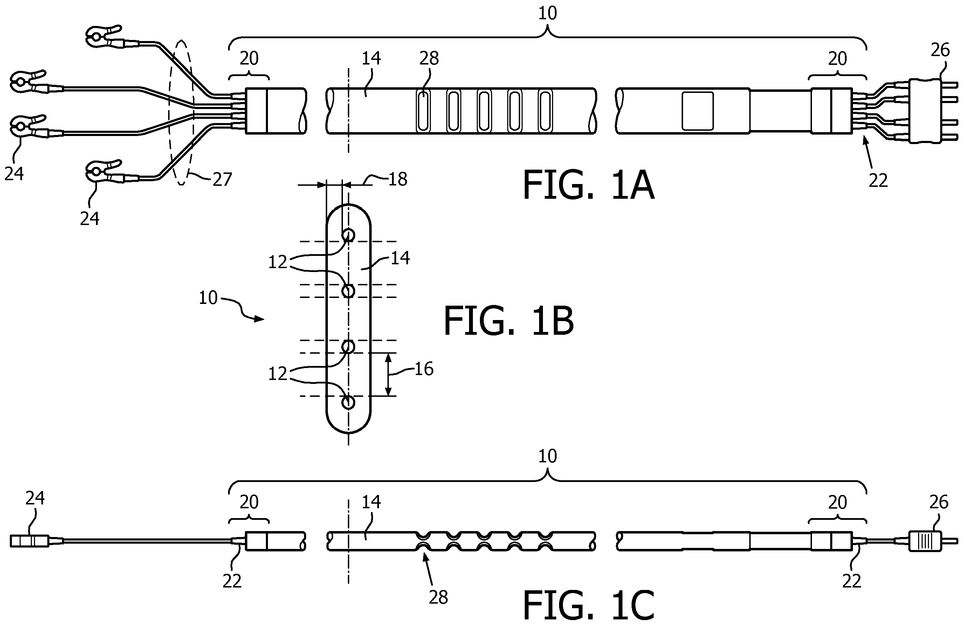

With reference to FIGURE 1A, an embodiment of a planar magnetic resonance (MR) safe cable **10** for biopotential measurements is schematically illustrated in a top view. In FIGURE 1B, the MR safe cable **10** is shown in a cross section view, and in FIGURE 1C, the MR safe cable **10** is shown in a side view. The cable **10** is shown with four controlled resistance electrically conductive wires **12** disposed in a common plane and parallel to each other. The cable can be configured in other embodiments to hold any number of wires, e.g., 4 to 12, in the common plane and parallel to each other. The wires transmit physiological signals such as ECG signals in a strong magnetic field. The strong magnetic field can be a magnetic field of a magnetic resonance imaging (MRI) scanner. The controlled resistance electrically conductive wires in one embodiment include a distance **16** of at least 4 mm between adjacent wires, but other distances are contemplated.

The cable **10** includes a stiff flat substrate **14** which holds the four or more controlled resistance conductive wires in the common plane and parallel to each other. The substrate is made of a non-proton emitting material. The stiff planar substrate can include a foam plastic jacket which surrounds and holds the conductive wires parallel and in the common plane. Holding the conductive wires parallel and in a common plane increases the likelihood that induced currents will be induced equally on equal wire and therefore can be filtered out more easily due to "common-mode". The foam jacket thermally and electrically insulates the wires from contact with the patient. The foam in one embodiment includes at least 2.5 mm of foam **18** between each controlled resistance electrically conductive wire and an outside surface. The foam can include open or closed cell foam. The closed cell foam provides easier clean-up.

End caps **20** attached to each end of the stiff planar substrate can provide strain relief to the four or more controlled resistance electrically conductive wires and guide the wires into predetermine connections. For example, each wire can be supported with strain relief **22** between a connector and an end of the substrate or foam jacket. The end caps can provide a rigid surface for grasping the cable and fitting the connector. The end caps reduce overall wear on the cable. Connectors **24** disposed at one end of the cable connect, e.g. clip, each wire to a contact of an ECG electrode. Connectors **26** disposed at another end connect, e.g. plug, the wires to a patient monitor. The connectors can be keyed such as color coded, marked, shaped, and the like, which provide a healthcare practitioner easy identification of corresponding predetermined connections for each connector. The connectors can be individual connectors such as shown with individual leads **27** and the electrode connectors, or unitary connector such as shown with the patient monitor connectors. A unitary connector can include webbing which additionally provides strain relief, organization, and ordered connection. The leads **27** can be in a staggered configuration as shown with varying length of leads or in a straight configuration with equal length leads.

The cabling can include necking **28** in the foam jacket or substrate to facilitate storage. For example, the cable can be wound in a large coil and fastened with a temporary fastener for storage. Removing the fasteners allows the cable to extend or return to the planar position.

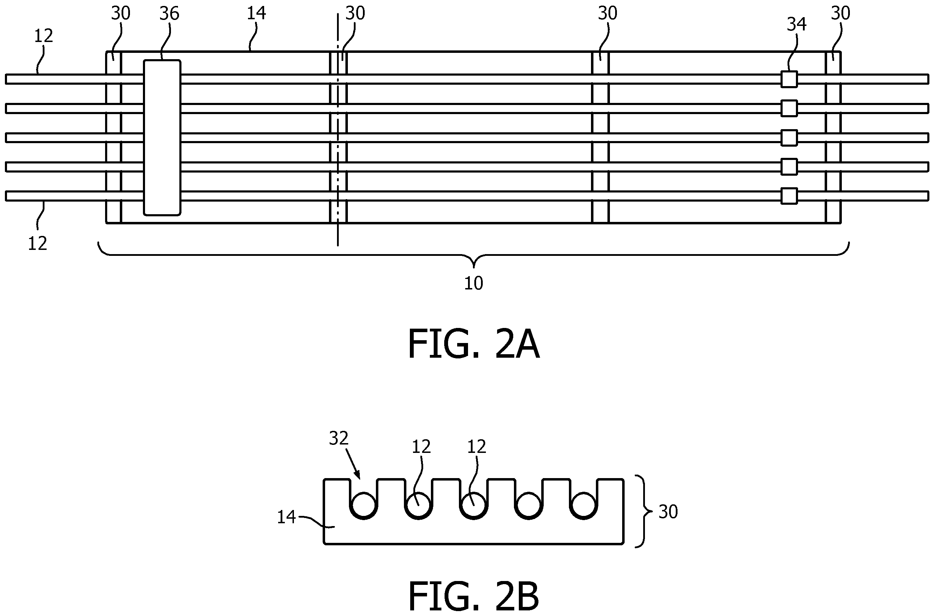

With reference to FIGURES 2A-2B, another embodiment of the planar magnetic resonance safe cable **10** for biopotential measurements is schematically illustrated. In FIGURE 2A, the cable **10** is shown in a top view, and in FIGURE 2B, the cable **10** is shown in a cross section view. As shown, the stiff planar substrate **14** holds five controlled resistance conductive wires **12** in a common plane and parallel to each other. The substrate **14** includes MR inert material or material that does not emit protons. The stiff substrate **14** includes a plurality of cable combs **30** spaced to hold the controlled resistance wires **12** in the common plane, with a fixed spacing and parallel to each other. Each comb **30** includes slots **32** and each slot holds one of the wires parallel to adjacent wires and the wires collectively in the common plane.

Each controlled resistance electrically conductive wire can include electric shock inhibiting components **34,** such as discrete resistors, RF chokes, and the like. For example, a predetermined or variable resistor is spliced into each wire to control resistance of the wire. The wires **12** can individually or collectively include one or more additional components **36** such as a notch filter, low pass filter, integrated circuit, sensor, and the like. The filters can block currents at frequencies other than the frequencies with ECG signals.

The embodiment of FIGURE 2A and 2B can be integrated into the embodiment of FIGURE 1A-1C such as using the cable combs during manufacture and integrating the substrate of FIGURE 2A and 2B into the foam jacket.

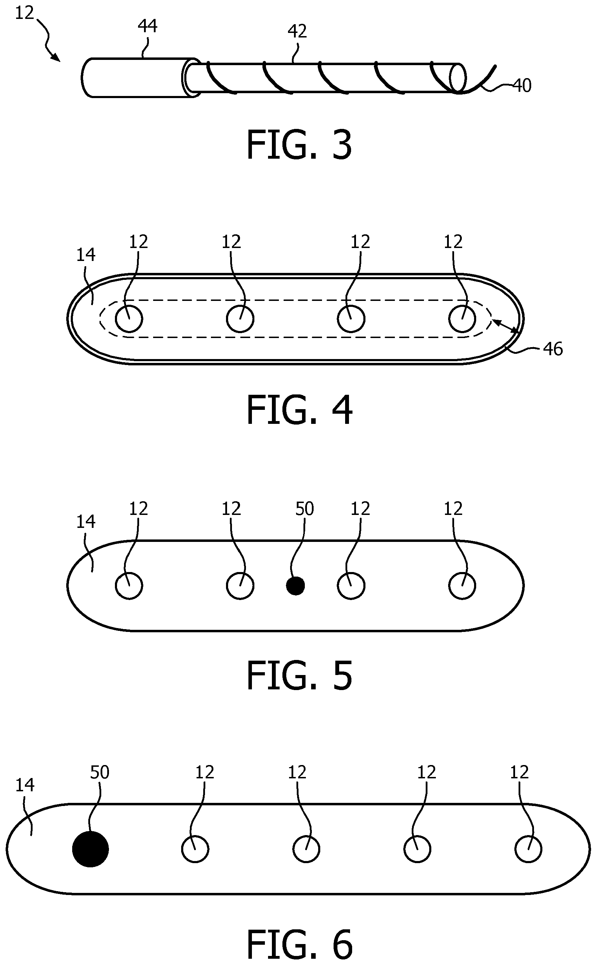

With reference to FIGURE 3, an embodiment of a controlled resistance electrically conductive wire **12** is schematically illustrated. The controlled resistance electrically conductive wire includes fine high resistance wire **40** wound spirally around a non-conductive core **42.** The fine high resistance wire **40** includes a nickel copper alloy. The controlled resistance electrically conductive wire **12** can include an insulating material cover **44.** The non-conductive core **42** and the insulating material cover **44** are made of MR inert material.

With reference to FIGURE 4, another embodiment of the planar magnetic resonance safe cable **10** is schematically illustrated in a cross section. The cable can include a protective shield **46,** such as a non-ferrous foil or copper mesh, surrounding the controlled resistance electrically conductive wires **12.** The protective shield further protects the wires **12** from electrical interference. The protective shield is disposed as an external covering as shown or integrated into the foam jacket as indicated by the dotted lines.

With reference to FIGURE 5 another embodiment of the planar magnetic resonance safe cable **10** is schematically illustrated in a cross section with an additional optically or electrically conductive component **50,** such as an optical fiber, antenna lead, sensor lead, integrated circuit, power supply lead, and the like. The conductive component can be located between the controlled resistance electrically conductive wires and/or as shown in FIGURE 6 adjacent to the wires **12.**

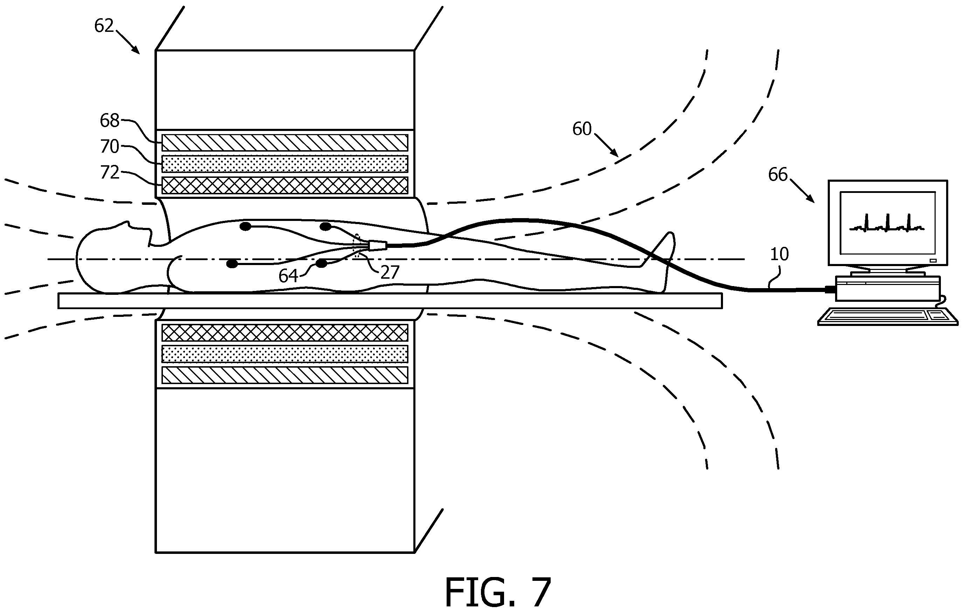

With reference to FIGURE 7 another embodiment of the planar magnetic resonance safe cable **10** schematically illustrated in a magnetic field **60** of an MR scanner **62.** The MR scanner **62** is shown in a cross section. The cable **10** is shown connected by leads **27** to electrodes **64** of a subject and to a patient monitor **66,** such as an ECG monitor, respiratory monitor, and the like. The MR scanner generates a horizontal or vertical static B₀ field from a magnet coil **68.** The MR scanner generates gradient magnetic or B₁ fields from gradient coils **70.** The MR scanner induces resonance in tissues of the subject with a local and/or whole body RF coil **72.** The cable **10** receives the sensed physiological signals from electrodes attached to the subject and transmits the physiological signals to the patient monitor in the presence of the magnetic and RF fields generated by the various coils.

The cable **10** holds the controlled resistance electrically conductive wires planar and parallel. The cable substrate forms a stiff jacket that resists looping and twisting. The substrate returns to the cable to a substantially straight cable run. The cable without force applied returns to a planar configuration, e.g. if placed on a flat surface will return to straight and flat.

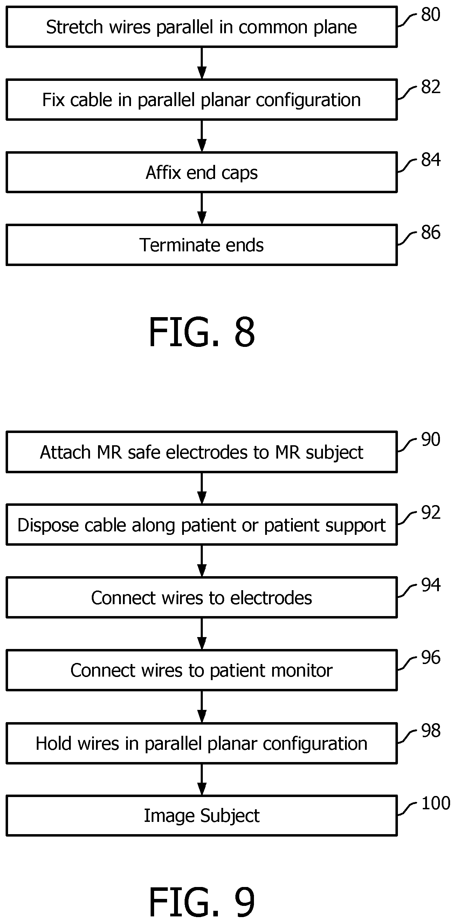

With reference to FIGURE 8, one method of manufacturing an embodiment of the planar magnetic resonance safe cable **10** for biopotential measurements is flowcharted. In a step **80** at least four controlled resistance electrically conductive wires **12** are stretched parallel and in a common plane. The wires are stretched perpendicular between two lines such as between two cable combs. The step can include stretching additional leads in the common plane such as optical fibers, antenna, power cables, and the like. The step can include adding discrete resistance components, integrated circuits, sensors, and the like.

In a step **82,** a stiff foam jacket coats the stretched at least four controlled resistance electrically conductive wires. The stiff foam jacket holds or fixes the wires parallel and in the common plane. In one embodiment, the foam jacket is pressure molded. The cable combs can be integrated into the stiff foam jacket.

Affixing end caps to each end of the foam jacket provide strain relief in a step **84.** The end caps can be integrated with the foam jacket or added as a separate step.

In a step **86,** the least four controller resistance electrically conductive wires are terminated at a first end with electrode connectors and at a second end with patient monitor connectors. The step can include adding keys to the connectors or leads.

With reference to FIGURE 9, one method of using an embodiment of the planar magnetic resonance safe cable **10** for biopotential measurements is flowcharted. In a step **90,** at least four electrodes are attached to a subject.

In a step **92,** the MR safe cable is disposed along the subject supported by the subject or a subject support. The MR safe cable includes the at least four controlled resistance conductive wires disposed in a parallel planar configuration. The step can include releasing the MR safe cable from a storage configuration to an operation configuration.

A connector attached to one of the corresponding connectors of a first end of the at least four controlled resistance conductive wires is connected to the electrodes in a step **94.** Connectors attached to a second end of the controlled resistance conductive wires are connected to a patient monitor in a step **96.**

With a stiff non-proton emitting or MR inert substrate of the MR safe cable which encases the controlled resistance wires in the parallel planar configuration, the four or more controlled resistance conductive wires are held in the planar and parallel configuration in a step **98.** The wires transmit the ECG signals from the electrodes to the patient monitor for biopotential meansurements. The stiff substrate can include the embodiments of the cable as described in reference to FIGURES 1A-1C, 2A-2B, 3-7, and combinations thereof.

In a step **100,** the subject can be imaged with a magnetic resonance scanner and the connected MR safe cable. The step includes transmitting physiological signals such as ECG signals with the MR safe cable concurrently with the imaging which are analyzed and/or displayed. The imaging can include processing of the transmitted signals such as with triggered or gated imaging.

It is to be appreciated that in connection with the particular illustrative embodiments presented herein certain structural and/or function features are described as being incorporated in defined elements and/or components. However, it is contemplated that these features may, to the same or similar benefit, also likewise be incorporated in other elements and/or components where appropriate. It is also to be appreciated that different aspects of the exemplary embodiments may be selectively employed as appropriate to achieve other alternate embodiments suited for desired applications, the other alternate embodiments thereby realizing the respective advantages of the aspects incorporated therein.

For example, the lead wires **12** can be disposed in other relationships that resist looping, twisting, or otherwise moving relative to each other. In one example, the lead wires are disposed alternately in first and second adjacent parallel planes.

It is also to be appreciated that particular elements or components described herein may have their functionality suitably implemented via hardware, software, firmware or a combination thereof. Additionally, it is to be appreciated that certain elements described herein as incorporated together may under suitable circumstances be stand-alone elements or otherwise divided. Similarly, a plurality of particular functions described as being carried out by one particular element may be carried out by a plurality of distinct elements acting independently to carry out individual functions, or certain individual functions may be split-up and carried out by a plurality of distinct elements acting in concert. Alternately, some elements or components otherwise described and/or shown herein as distinct from one another may be physically or functionally combined where appropriate.

In short, the present specification has been set forth with reference to preferred embodiments. Obviously, modifications and alterations will occur to others upon reading and understanding the present specification. It is intended that the invention be construed as including all such modifications and alterations insofar as they come within the scope of the appended claims or the equivalents thereof. That is to say, it will be appreciated that various of the above-disclosed and other features and functions, or alternatives thereof, may be desirably combined into many other different systems or applications, and also that various presently unforeseen or unanticipated alternatives, modifications, variations or improvements therein may be subsequently made by those skilled in the art.

## Claims

1. A magnetic resonance (MR) safe cable (10), comprising:
four or more controlled resistance electrically conductive wires (12) disposed in a planar configuration and parallel to each other, wherein the controlled resistance electrically conductive wires include fine high resistance wire (40) wound spirally around a non-conductive core (42) and wherein the fine high resistance wire (40) includes a nickel copper alloy, and
a stiff non-proton emitting substrate (14) which holds the four or more controlled resistance conductive wires in the planar configuration and parallel to each other,
wherein the stiff substrate (14) includes a flat foam jacket which surrounds and holds the conductive wires parallel and in the common plane, and
wherein the stiff substrate (14) includes a plurality of longitudinally spaced cable combs (30) spaced to hold the controlled resistance wires (12) in the planar configuration and parallel to each other, each comb including a plurality of slots (32) configured to receive and anchor one of the wires in the parallel planar configuration.

2. The MR safe cable (10) according to claim 1, wherein the stiff substrate (14) fixes the controlled resistance electrically conductive wires (12) in the common plane separated by at least 4 mm.

3. The MR safe cable (10) according to any one of claims 1-2, wherein the stiff substrate (14) includes closed cell foam.

4. The MR safe cable (10) according to any one of claims 1-3, wherein the stiff substrate (14) includes at least 2.5 mm of foam between each controlled resistance electrically conductive wire and an outside surface.

5. The MR safe cable (10) according to any one of claims 1-4, wherein each controlled resistance electrically conductive wire (12) is covered with an insulating material (44).

6. The MR safe cable (10) according to any one of claims 1-5, further including:
a protective shield (46) surrounding the stiff substrate (14).

7. A method of using a magnetic resonance (MR) safe cable, comprising:
attaching (90) at least four electrodes to a subject;
disposing (92) the MR safe cable along the subject supported by the subject or a subject support, the MR cable including at least four controlled resistance conductive wires (12) disposed in a parallel planar configuration, wherein the controlled resistance electrically conductive wires include fine high resistance wire (40) wound spirally around a non-conductive core (42) and wherein the fine high resistance wire (40) includes a nickel copper alloy;
connecting (94) a connector attached to one of the corresponding connectors of a first end of the at least four controlled resistance conductive wires to the electrodes;
connecting (96) connectors attached to a second end of the controlled resistance conductive wires to a patient monitor; and
with a stiff non-proton emitting substrate (14) of the MR safe cable (10) including a flat foam jacket which surrounds and holds the controlled resistance conductive wires in the parallel planar configuration and wherein the stiff substrate (14) includes a plurality of longitudinally spaced cable combs (30) spaced to hold the controlled resistance wires (12) in the planar configuration and parallel to each other, each comb including a plurality of slots (32) configured to receive and anchor one of the wires in the parallel planar configuration; and
holding (98) the four or more controlled resistance conductive wires in the planar and parallel configuration.

8. The method according to claim 7, further including:
imaging (100) the subject with a magnetic resonance scanner with the MR safe cable disposed partially in an imaging region of the magnetic resonance scanner.

9. A method of manufacturing a magnetic resonance (MR) safe cable, comprising:
stretching (80) at least four controlled resistance electrically conductive wires parallel and in a plane, wherein the controlled resistance electrically conductive wires include fine high resistance wire (40) wound spirally around a non-conductive core (42) and wherein the fine high resistance wire (40) includes a nickel copper alloy;
coating (82) the stretched at least four controlled resistance electrically conductive wires with a stiff substrate including a stiff foam jacket and a plurality of longitudinally spaced cable combs (30), wherein the stiff foam jacket surrounds and holds the conductive wires parallel and in the plane, and the plurality of longitudinally spaced cable combs (30) are spaced to hold the controlled resistance wires (12) in the planar configuration and parallel to each other, each comb including a plurality of slots (32) to receive and anchor one of the wires in the parallel planar configuration.

## Patentansprüche

1. Ein Magnetresonanz-(MR)-Sicherheitskabel (10), das Folgendes umfasst:
vier oder mehr elektrisch leitende Drähte (12) mit gesteuertem Widerstand, die
in einer planaren Konfiguration und parallel zueinander angeordnet sind, wobei die
elektrisch leitenden Drähte einen feinen Hochwiderstandsdraht (40) umfassen, der spiralförmig um einen nichtleitenden Kern (42) gewickelt ist, und wobei der feine Hochwiderstandsdraht (40) eine Nickelkupferlegierung enthält, und
ein steifes, keine Protonen emittierendes Substrat (14), das die vier oder mehr leitenden Drähte mit kontrolliertem Widerstand in der planaren Konfiguration und parallel zueinander hält,
wobei das steife Substrat (14) einen flachen Schaumstoffmantel aufweist
und die leitenden Drähte parallel und in der gemeinsamen Ebene hält, und wobei das steife Substrat (14) eine Vielzahl von in Längsrichtung beabstandeten Kabelkämme (30) enthält, die so beabstandet sind, dass sie die Drähte (12) mit gesteuertem Widerstand in der planaren Konfiguration und parallel zueinander halten,
wobei jeder Kamm eine Vielzahl von Schlitzen (32) aufweist die so konfiguriert sind, dass sie einen der Drähte in der parallelen planaren Konfiguration aufnehmen und verankern.

2. MR-Sicherheitskabel (10) nach Anspruch 1, wobei das steife Substrat (14)
die elektrisch leitenden Drähte (12) mit kontrolliertem Widerstand in der gemeinsamen Ebene mit einem Abstand von mindestens 4 mm fixiert.

3. MR-Sicherheitskabel (10) nach einem der Ansprüche 1-2, wobei das steife Substrat (14) einen geschlossenzelligen Schaumstoff enthält.

4. MR-Sicherheitskabel (10) nach einem der Ansprüche 1-3, wobei das steife Substrat (14) mindestens 2,5 mm Schaumstoff zwischen
jedem elektrisch leitenden Draht mit kontrolliertem Widerstand und einer Außenfläche enthält.

5. MR-Sicherheitskabel (10) nach einem der Ansprüche 1-4, wobei jeder elektrisch leitende Draht mit kontrolliertem Widerstand (12) mit einem isolierenden Material überzogen ist (44).

6. Das MR-Sicherheitskabel (10) nach einem der Ansprüche 1-5, das außerdem Folgendes umfasst:
eine Schutzabschirmung (46), die das steife Substrat (14) umgibt.

7. Verfahren zur Verwendung eines sicheren Kabels für magnetische Resonanz (MR), das Folgendes umfasst:
Anbringen (90) von mindestens vier Elektroden an einer Person; Anordnen (92) des MR-Sicherheitskabels entlang des vom Patienten oder einer Tesperson getragenen Objekt, wobei das MR-Kabel mindestens vier leitende Drähte mit kontrolliertem Widerstand (12) enthält, die in einer parallelen planaren Konfiguration angeordnet sind, wobei die elektrisch leitenden Drähte mit kontrolliertem Widerstand
einen feinen Hochwiderstandsdraht (40) umfassen, der spiralförmig um einen nicht-leitenden Kern (42) gewickelt sind und wobei der feine Hochwiderstandsdraht (40) eine Nickel-Kupfer Legierung enthält;
Verbinden (94) eines Verbinders, der an einem der entsprechenden Verbinder eines ersten Endes der mindestens vier leitenden Drähte mit kontrolliertem Widerstand mit den Elektroden angehängt ist;
Verbinder (96), die an einem zweiten Ende der kontrollierten Widerstandsleitdrähte mit einem Patientenmonitor angehängt sind; und
mit einem steifen, keine Protonen emittierenden Substrat (14) des MR-sicheren Kabels (10)
einschließlich eines flachen Schaumstoffmantels, der die die leitenden Drähte mit kontrolliertem Widerstand in der parallelen planaren Konfiguration umgibt und hält und wobei das steife Substrat (14)
eine Vielzahl von in Längsrichtung beabstandeten Kabelkämmen (30), die beabstandet sind, um die Drähte (12) mit gesteuertem Widerstand in der planaren Konfiguration und parallel zueinander zu halten parallel zueinander zu halten, wobei jeder Kamm eine Vielzahl von Schlitzen (32) aufweist, die so konfiguriert sind, dass sie einen der
der Drähte in der parallelen planaren Konfiguration aufzunehmen und zu verankern; und
Halten (98) der vier oder mehr leitenden Drähte mit kontrolliertem Widerstand in der planaren und parallelen Konfiguration.

8. Das Verfahren nach Anspruch 7, das ferner Folgendes umfasst:
Bildgebung (100) des Patienten mit einem Magnetresonanztomographen, wobei das MR-Safe Kabel teilweise in einem Bildgebungsbereich des Magnetresonanztomographen angeordnet ist.

9. Verfahren zur Herstellung eines für magnetische Resonanz (MR) Sicherheitskabels, das Folgendes umfasst:
Strecken (80) von mindestens vier elektrisch leitenden Drähten mit kontrolliertem Widerstand parallel und in einer Ebene, wobei die elektrisch leitenden Drähte mit kontrolliertem Widerstand einen feinen Hochwiderstandsdraht (40) umfassen, der spiralförmig um einen nichtleitenden Kern (42) gewickelt ist, und wobei der feine Hochwiderstandsdraht (40) eine Nickel-KupferLegierung enthält;
Beschichten (82) der gestreckten mindestens vier elektrisch leitenden Drähte mit kontrolliertem Widerstand mit einem steifen Substrat, das einen steifen Schaumstoffmantel und eine Vielzahl von Kabelkämmen (30) enthält, wobei der steife Schaumstoffmantel die leitenden Drähte parallel und in der Ebene umgibt und hält und die Mehrzahl der in Längsrichtung beabstandeten Kabelkämme (30) beabstandet sind, um die Drähte (12) mit gesteuertem Widerstand in der planaren Konfiguration und parallel zueinander zu halten, wobei jeder Kamm eine Vielzahl von Schlitzen (32) zur Aufnahme und Verankerung einen der Drähte in der parallelen, ebenen Konfiguration.

## Revendications

1. Une résonance magnétique (MR) d'un câble de sécurité (10), comprend:
quatre ou plusieurs fils conducteurs d'électricité à résistance contrôlée (12) disposés dans une configuration planaire et parallèle l'un par rapport à l'autre, où les fils conducteurs d'électricité à résistance contrôlée inclut un fil fin à haute résistance (40) enroulé en spirale autour d'un noyau non conducteur (42) et où le fil fin à haute résistance (40) inclut un alliage de nickel et de cuivre, et un substrat rigide non émetteur de protons (14) qui maintient quatre ou plusieurs fils conducteurs d'électricité à résistance contrôlée dans une configuration planaire et parallèle l'un à l'autre, où le substrat rigide (14) inclut une gaine plate en mousse qui entoure et maintient les fils conducteurs parallèles et dans un plan commun, et où le substrat rigide (14) inclut une pluralité de rayons de câbles espacés longitudinalement (30) espacés pour maintenir les fils conducteurs à résistance contrôlée (12) dans une configuration planaire et parallèle l'un à l'autre, chaque peigne inclut une pluralité d'emplacements (32) configurés pour recevoir et ancrer un des fils dans la configuration planaire parallèle.

2. Le câble de sécurité MR (10) selon la revendication 1, où le substrat rigide (14) fixe les fils conducteurs à résistance contrôlée (12) dans le plan commun séparés par au moins 4 mm.

3. Le câble de sécurité MR (10) selon l'une quelconque des revendications 1-2, où le substrat rigide (14) inclut une mousse à cellule fermée.

4. Le câble de sécurité MR (10) selon l'une quelconque des revendications précédentes 1-3, où le substrat rigide (14) inclut au moins 2,5 mm de mousse entre chaque fils conducteur à résistance électrique et une surface extérieure.

5. Le câble de sécurité MR (10) selon l'une quelconque des revendications précédentes 1-4, où chaque fils électrique conducteur à résistance contrôlé (12) est couvert avec un matériau isolant (44).

6. Le câble de sécurité MR (10) selon l'une quelconque des revendications précédentes 1-5 comprend également:
un bouclier de protection (46) entourant le substrat rigide (14).

7. Une méthode d'utilisation d'une résonance magnétique (MR) un câble de sécurité, comprenant:
l'attache (90) à au moins quatre électrodes chez un sujet;
la disposition (92) d'un câble de sécurité MR le long du sujet soutenu par le sujet ou le support du sujet, le câble MR inclut au moins quatre fils conducteurs à résistance contrôlée (12) disposés dans une configuration planaire parallèle, où les fils conducteurs à résistance électrique contrôlée inclut un fil fin à haute résistance (40) enroulé en spirale autour d'un noyau non conducteur (42) et où le fil fin à haute résistance (40) inclut un alliage de nickel cuivre;
la connexion (94) d'un connecteur attaché à un des connecteurs correspondants d'une première extrémité d'au moins quatre fils conducteurs de résistance contrôlée à des électrodes;
la connexion (96) de connecteurs attachés à une seconde extrémité de fils conducteurs de résistance contrôlée à un moniteur de patient; et
avec un substrat rigide de non-émission de protons (14) du câble de sécurité MR (10) incluant une gaine plate en mousse qui entoure et maintient les fils conducteurs de résistance contrôlée dans une configuration planaire parallèle et où le substrat rigide (14) inclut une pluralité de câbles de peignes longitudinalement espacés (30) espacés pour maintenir les fils de résistance contrôlés (12) dans la configuration planaire et
parallèle l'un par rapport à l'autre, chaque peigne inclut une pluralité d'espacements (32) configurés pour recevoir et ancrer un des fils dans la configuration planaire parallèle; et
le maintien (98) de quatre ou plusieurs fils conducteurs de résistance contrôlée dans une configuration planaire et parallèle.

8. La méthode selon la revendication 7 comprend également:
l'imagerie (100) du sujet avec un scanner de résonance magnétique avec un câble de sécurité MR disposé partiellement dans une région d'imagerie du scanner de résonance magnétique.

9. Une méthode de fabrication un câble sécurisé par résonance magnétique (MR), comprend:
l'étirement (80) d'au moins quatre fils conducteurs électriques de résistance contrôlée parallèle et dans un plan, où les fils électriques à résistance contrôlée inclut un fil fin à haute résistance (40) enroulé en spirale autour d'un noyau non conducteur (42) et où le fil fin à haute résistance (40) inclut un alliage nickel cuivre; le revêtement (82) des fils étirés d'au moins quatre résistances électriques contrôlées avec un substrat rigide comprenant une gaine rigide en mousse et une pluralité de câbles de peignes longitudinalement espacés (30), où une gaine rigide en mousse entoure et maintient les fils conducteurs parallèles et dans le plan, et la pluralité de câbles de peignes longitudinalement espacés (30) sont espacés pour maintenir les fils de résistance contrôlée (12) dans une configuration planaire et parallèle l'un à l'autre, chaque peigne inclut une pluralité d'espacements (32) pour recevoir et ancrer un des fils dans une configuration planaire parallèle.
